# FASCICULE DE BREVET EUROPEEN

(11) **EP 1 563 293 B1**
(45) Date de publication et mention de la délivrance du brevet: **28.11.2007**
(21) Numéro de dépôt: 03767856.2
(22) Date de dépôt: 29.10.2003
(51) Int. Cl.: G01N 33/28

(54) **PROCEDE DE DETERMINATION DU POINT DE DISPARITION DES CRISTAUX DE PRODUITS PETROLIERS AINSI QUE DISPOSITIF PERMETTANT LA MISE EN OEUVRE DE CE PROCEDE**
VERFAHREN ZUR BESTIMMUNG DER VERFLÜCHTIGUNGSTEMPERATUR VON ERDÖLPRODUKTKRISTALLEN UND EINRICHTUNG DAFÜR
METHOD FOR DETERMINING VANISHING TEMPERATURE OF PETROLEUM PRODUCT CRYSTALS AND DEVICE THEREFOR

(30) Priorité: 30.10.2002 FR 0213577
(43) Date de publication de la demande: 17.08.2005
(73) Titulaire: I.S.L., 14790 Verson (FR)
(72) Inventeur: CLERIS, Hervé, F-14220 Curcy sur Orne (FR); LARA, Olivier, F-86130 Jaunay-Clan (FR)
(74) Mandataire: Cabinet HERRBURGER
(86) Numéro de dépôt international: PCT/FR2003/003222
(87) Numéro de publication internationale: WO 2004/042385

(56) Documents cités:
- EP-A- 0 328 334
- DE-A- 10 056 131
- US-A- 3 457 772
- US-A- 4 519 717

## Description

La présente invention concerne un procédé de détermination du point de disparition des cristaux de produits pétroliers, en particulier de kérosènes destinés à l'aviation dans une gamme de températures d'environ -5 à -120°C.

Le point de disparition des cristaux est défini comme la température de disparition des derniers cristaux dans un échantillon préalablement cristallisé, au cours d'une remontée graduelle de la température.

Il existe diverses normes définissant les conditions d'obtention du point de disparition des cristaux ; celui-ci présente un intérêt tout particulier pour les spécialistes du domaine de l'aviation, vu qu'il permet de déterminer le temps pendant lequel un avion peut rester à une haute altitude donnée sans risque d'obturation des conduites de transfert du carburant et des filtres.

De plus, la valeur du point de disparition des cristaux permet de savoir si un kérosène est pur ou pollué par du gasoil.

Il existe actuellement sur le marché différents appareils permettant de déterminer le point de disparition des cristaux d'échantillons de produits pétroliers.

Parmi ces appareils, on peut à titre d'exemple mentionner les appareils commercialisés par la Société ISL sous les dénominations FZP 5 Gs et FZP 5 G qui sont des appareils totalement automatisés fonctionnant en application des normes ASTM D 2386, IP 468 et ISO 3013, ou bien encore l'appareil décrit dans le document US-4519 717.

Le principe de tels appareils consiste schématiquement à faire passer un faisceau lumineux émis par une diode au travers d'une cellule de mesure renfermant l'échantillon à analyser qui est placée dans une chambre cryostatée équipée d'un capteur de température relié à des organes de réfrigération et de réglage de la température, et à détecter l'intensité lumineuse reçue par un récepteur optique aligné sur l'émetteur infrarouge au travers de la cellule de mesure renfermant les échantillons à analyser.

Pour mettre en oeuvre ce test, on abaisse graduellement la température de la chambre cryostatée jusqu'à ce que le détecteur optique ne reçoive plus de lumière, ce qui signifie que l'échantillon est totalement cristallisé, puis on augmente à nouveau graduellement la température tout en enregistrant la courbe représentant les variations de l'intensité lumineuse reçue par le récepteur optique en fonction de la température.

Lorsque les derniers cristaux ont disparu dans l'échantillon, on observe sur cette courbe, une cassure qui correspond au point de disparition des cristaux suivie d'un palier.

Un tel appareil présente l'avantage d'être compact et automatique, donc de pouvoir donner des résultats parfaitement reproductibles sans dépendre de l'habileté d'un opérateur.

Il souffre toutefois de l'inconvénient d'avoir une sensibilité pouvant s'avérer insuffisante dans certains cas, et en particulier lorsque l'on cherche à déterminer le point de disparition des cristaux d'un kérosène pollué par une faible proportion de gasoil.

En effet, en présence de gasoil, le point de disparition des cristaux d'un échantillon de kérosène augmente notablement : dans le cas d'un échantillon renfermant quelques % de gasoil, la courbe représentant les variations de l'intensité reçue par le détecteur optique en fonction de la température présente une cassure suffisamment nette pour déterminer le point de disparition des cristaux, et par suite la proportion de gasoil par comparaison avec le point de disparition des cristaux du kérosène pur.

Au contraire, dans le cas d'une pollution de proportion moindre, la courbe s'arrondit et ne présente plus de cassure nette de sorte qu'il n'est plus possible de déterminer le point de disparition des cristaux.

Un autre appareil adapté à la détermination du point de disparition des cristaux est décrit dans le brevet US-5 088 833.

Le principe de cet appareil qui fonctionne selon la norme ASTM D 5972 consiste schématiquement à déposer un micro échantillon du produit à analyser dans une coupelle dont le fond est constitué par un miroir refroidi par des éléments Peltier, et à refroidir graduellement cet échantillon jusqu'à sa cristallisation avant de le réchauffer à nouveau graduellement.

Durant l'essai, l'échantillon à analyser est éclairé par un faisceau lumineux avec une incidence choisie de sorte que le faisceau réfléchi sur le miroir n'atteigne pas un détecteur optique situé au-dessus de celui-ci.

Lorsque des cristaux sont présents dans l'échantillon, ils diffusent de façon aléatoire la lumière émise et par suite une partie de cette lumière est reçue par le détecteur optique.

En conséquence, l'apparition et la disparition des cristaux peuvent être détectées par l'analyse du signal reçu par le détecteur optique qui est nul en l'absence de cristaux et augmente avec l'apparition de cristaux dans l'échantillon.

Cet appareil présente l'avantage d'avoir une sensibilité suffisante pour permettre de détecter une très faible quantité de gasoil au sein d'un kérosène. Son utilisation est toutefois peu commode, et les résultats obtenus sont dans une large mesure dépendants de l'habileté de l'opérateur. L'utilisation d'un récepteur optique latéral est connu de EP-0328334.

La présente invention a pour objet de proposer un procédé de détermination du point de disparition des cristaux de produits pétroliers, en particulier de kérosènes destinés à l'aviation, de nature à remédier à ces inconvénients.

Selon l'invention, ce procédé est caractérisé par les étapes suivantes :
- on monte un émetteur laser ainsi qu'un récepteur optique longitudinal associé de part et d'autre d'une cellule de mesure tubulaire essentiellement horizontale placée dans une chambre cryostatée équipée d'un capteur de température relié à des organes de réfrigération et de réglage de la température de sorte que le faisceau optique émis par l'émetteur laser soit aligné sur l'axe longitudinal de la cellule de mesure et sur le récepteur optique longitudinal,
- on relie le capteur de température, les organes de réfrigération et de réglage de la température, ainsi que le récepteur optique longitudinal à des moyens de calcul et d'affichage programmables,
- on monte un diaphragme directement en aval de l'émetteur laser de sorte que le faisceau optique émis par celui-ci soit suffisamment fin pour exclure toute réflexion sur les parois de la cellule de mesure,
- on monte, en amont du récepteur optique longitudinal, un polariseur règle de façon à ne pas pouvoir transmettre le faisceau optique directement émis par émetteur laser,
- on introduit l'échantillon à analyser dans la cellule de mesure,
- on branche l'émetteur laser et le récepteur optique longitudinal associé de façon à faire passer un faisceau optique au travers de l'échantillon à analyser et on enregistre l'intensité lumineuse reçue par le récepteur optique longitudinal,
- on abaisse graduellement la température de la chambre cryostatée jusqu'à la température de fin de cristallisation de l'échantillon à analyser ou point d'opacité puis on augmente à nouveau graduellement la température de cette chambre en enregistrant la courbe représentant les variations de l'intensité lumineuse reçue par le récepteur optique longitudinal en fonction de la température ou courbe de détection, et
- on détermine le point de disparition des cristaux à partir de cette courbe.
   Ce procédé se distingue donc essentiellement par l'utilisation d'un faisceau de lumière polarisée de sorte que le récepteur optique longitudinal ne reçoive aucune lumière en l'absence de cristaux, alors qu'au contraire, dès l'apparition de cristaux au sein de l'échantillon à analyser une certaine quantité de lumière est transmise à ce récepteur ; il est en effet bien connu des spécialistes que les cristaux modifient la direction de polarisation de la lumière.

Un tel procédé ne peut bien entendu fonctionner qu'en l'absence de toute réflexion sur les parois de la cellule de mesure ; par suite, l'état de surface de cette cellule est indifférent, mais il est impératif que la section du faisceau traversant celle-ci soit suffisamment réduite par le diaphragme.

Conformément à l'invention on a pu établir que le diamètre du diaphragme doit de préférence être de l'ordre de 1 à 1,5 mm, vu qu'en de çà de 1 mm, on peut se heurter à des risques de diffraction à ce niveau.

En outre, pour obtenir une sensibilité optimum du récepteur, la longueur d'onde du faisceau laser peut avantageusement être de l'ordre de 650 nanomètres.

Selon une caractéristique préférentielle de l'invention, on monte également, à proximité de la cellule de mesure, à la partie amont de celle-ci, un récepteur optique latéral relié au faisceau optique émis par l'émetteur laser ainsi qu'aux moyens de calcul et d'affichage programmables.

Le récepteur optique latéral ne reçoit aucune lumière en l'absence de cristaux vu que l'échantillon à analyser est alors parfaitement transparent, mais reçoit de la lumière diffusée dès l'apparition de cristaux au sein de celui-ci.

Pendant la durée d'un test, on enregistre ainsi également la courbe représentant les variations de l'intensité lumineuse reçue par le récepteur optique latéral en fonction de la température ou courbe d'opacité et on détermine en utilisant cette courbe la température de fin de cristallisation de l'échantillon à analyser ou point d'opacité c'est-à-dire la température à partir de laquelle le sens de variation de la température doit être inversé.

Par suite, la fonction du récepteur optique latéral consiste à piloter le procédé.

D'une manière plus précise, au début du test, les deux détecteurs ne reçoivent aucune lumière.

En cours de refroidissement des premiers cristaux apparaissent qui modifient la polarisation de la lumière émise par l'émetteur laser et une certaine quantité de lumière peut ainsi traverser le polariseur et atteindre le récepteur optique longitudinal.

Lorsque la quantité de cristaux au sein de l'échantillon à analyser devient importante, celui-ci se trouble, provoquant ainsi une diffusion de la lumière dont une partie atteint le récepteur optique transversal.

Lorsque le trouble devient très important, le faisceau émis par l'émetteur laser ne peut plus atteindre le polariseur et par suite l'intensité lumineuse reçue par le détecteur optique longitudinal diminue.

Le point d'opacité est atteint lorsque l'intensité lumineuse reçue par le récepteur optique latéral augmente alors que l'intensité lumineuse reçue par le récepteur optique longitudinal diminue.

Lorsque le point d'opacité est atteint, on élève graduellement la température de la chambre cryostatée pour déterminer la valeur du point de disparition des cristaux de l'échantillon sur la courbe de détection.

Au cours de cette élévation, l'intensité lumineuse reçue par le récepteur optique longitudinal augmente à partir du moment où l'échantillon devient suffisamment transparent pour que le faisceau émis par l'émetteur laser puisse atteindre le polariseur puis diminue à nouveau lorsque les derniers cristaux disparaissent.

Le point à partir duquel le récepteur optique longitudinal ne reçoit plus aucune lumière correspond au point de disparition des cristaux recherché.

L'invention concerne également un dispositif permettant la mise en oeuvre du procédé susmentionné.

Selon l'invention, ce dispositif est caractérisé en ce qu'il comporte :
- une chambre cryostatée équipée d'un capteur de température relié à des organes de réfrigération et de réglage de la température,
- une tubulure de mesure essentiellement en forme de U montée à la partie interne de la chambre cryostatée et dont la branche centrale, essentiellement horizontale, constitue la cellule de mesure tandis que les branches latérales permettent l'introduction de l'échantillon à analyser dans cette cellule ainsi que son extraction,
- un émetteur laser et un récepteur optique longitudinal associé, alignés de part et d'autre de la cellule de mesure, le long de l'axe longitudinal de celle-ci,
- un diaphragme monté directement en aval de l'émetteur laser,
- un polariseur, monté en amont du récepteur optique longitudinal, et
- des moyens de calcul et d'affichage programmables reliés au capteur de température, aux organes de réfrigération et de réglage de la température, ainsi qu'au récepteur optique longitudinal.
   Compte tenu de cette configuration, les seules opérations manuelles devant être effectuées pour la mise en oeuvre d'un test consistent à introduire l'échantillon à analyser au moyen d'une seringue dans la cellule de mesure, et à brancher l'émetteur laser le récepteur optique longitudinal associé ainsi que les organes de réfrigération et de réglage de la température.

Le test s'effectue ensuite automatiquement sous le contrôle des moyens de calcul et d'affichage préalablement programmés en fonction de la norme à respecter qui commandent les organes de réfrigération et de réglage de la température en fonction des informations qui leur sont transmises par le capteur de température et établissent simultanément la courbe de détection en fonction des informations qui leur sont transmises par le récepteur optique longitudinal.

Selon une caractéristique préférentielle de l'invention, le dispositif comporte un récepteur optique latéral monté à proximité de la cellule de mesure, à la partie amont de celle-ci, et relié aux moyens de calcul et d'affichage programmables.

Conformément à cette caractéristique les moyens de calcul et d'affichage programmables établissent la courbe d'opacité à partir des informations qui leur sont transmises par le récepteur optique latéral et utilisent cette courbe pour piloter automatiquement les organes de réfrigération et de réglage de la température et par suite les variations de la température dans la chambre cryostatée.

Selon l'invention, l'intensité lumineuse est transmise aux récepteurs optiques par l'intermédiaire de guides de lumière coopérant de préférence avec des lentilles susceptibles de concentrer le faisceau optique.

Ces lentilles peuvent être avantageusement constituées par des billes de verre de 5 à 8 mm de diamètre situées dans l'axe optique.

Les guides de lumière sont quant à eux de préférence constitués par des fibres situées dans le plan focal de la lentille.

Selon une autre caractéristique de l'invention, la tubulure de mesure est constituée par un élément métallique, notamment réalisé en aluminium équipé de hublots permettant le passage du faisceau optique à détecter.

Il est essentiel que ces hublots qui sont en règle générale en verre aient des faces parfaitement parallèles.

Selon l'invention, les organes de réfrigération et de réglage de la température peuvent être constitués par une unité de refroidissement notamment à cycle de Stirling dont le doigt froid est équipé à son extrémité libre, d'organes de transmission de la chaleur à contact sec coopérant avec la chambre cryostatée de façon à permettre de la refroidir à la température souhaitée.

Un dispositif d'analyse d'échantillons de produits pétroliers renfermant une unité de refroidissement à cycle de Stirling est à titre d'exemple décrit dans le document FR-2 801 381.

La mise en oeuvre d'une telle unité de refroidissement correspond à une caractéristique particulièrement avantageuse de l'invention grâce à laquelle le dispositif peut être constitué par un appareil compact portatif.

Les caractéristiques du procédé et du dispositif conformes à l'invention seront décrites plus en détail en se référant aux dessins annexés dans lesquels :
- la figure 1 est un schéma représentant le dispositif
- les figures 2, 3 et 4 sont des exemples de courbes établies par les moyens de calcul et d'affichage programmables respectivement dans le cas d'un échantillon de kérosène pur, d'un échantillon de kérosène faiblement pollué et d'un échantillon de kérosène fortement pollué.

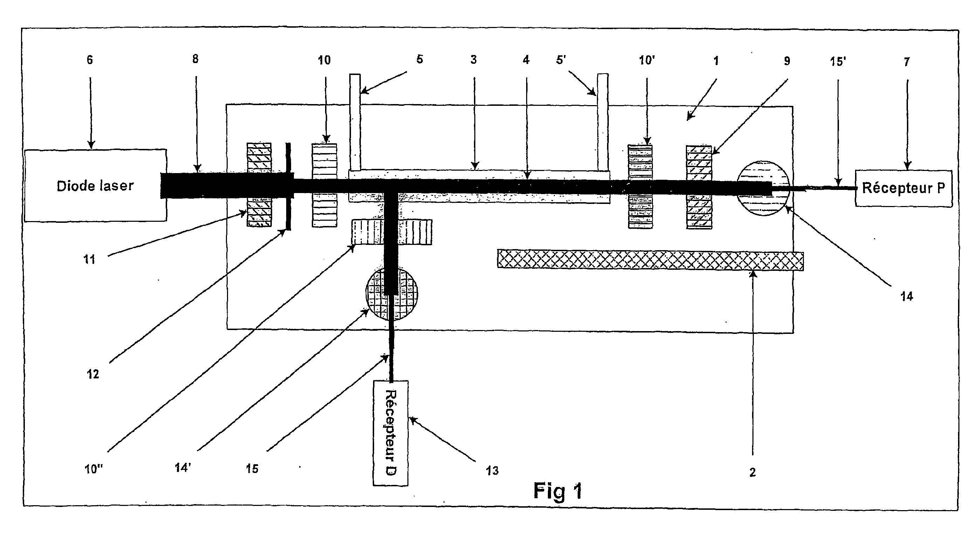

Selon la figure 1, le dispositif correspond à un appareil compact renfermant une chambre cryostatée 1 équipée d'un capteur de température 2 ainsi qu'une unité de réfrigération et de réglage de la température de cette chambre 1, à cycle de Stirling, non représentée sur la figure.

Selon la figure 1 la chambre cyostatée 1 est équipée, à sa partie interne d'une tubulure de mesure métallique en forme de U3, dont la branche centrale horizontale 4 constitue une cellule de mesure recevant l'échantillon à analyser.

Les branches latérales 5 et 5' de la tubulure de mesure 3 permettent l'introduction de cet échantillon ainsi que son évacuation.

L'appareil renferme également une diode laser 6 associée à un récepteur optique longitudinal 7 de sorte que le faisceau laser émis 8 soit aligné sur l'axe longitudinal de la cellule de mesure 4, et traverse l'échantillon à analyser introduit dans cette cellule avant de parvenir au récepteur 7.

Un polariseur 9 est monté en amont du récepteur longitudinal 7 dans le sens de propagation du faisceau laser émis par la diode 6.

Le polariseur 9 est réglé de sorte que le récepteur longitudinal 7 ne reçoive aucune lumière lorsque l'échantillon renfermé dans la cellule de mesure 4 est transparent et ne contient aucun cristaux.

Des hublots en verre 10, 10' ayant des faces parfaitement parallèles permettent au faisceau laser 8 de traverser la cellule de mesure 4 et de parvenir au récepteur longitudinal 7, tout en garantissant l'étanchéité de cette cellule.

Un polariseur auxiliaire 11 croisé avec le polariseur 9 et monté directement en aval de la diode laser 6 fait office d'atténuateur de l'amplitude du faisceau émis par cette diode.

Ce polariseur auxiliaire 11 coopère avec un diaphragme 12 monté directement en aval de celui-ci de façon à garantir que le faisceau laser traversant la cellule de mesure 4 soit suffisamment fin pour exclure toute réflexion sur les parois de cette cellule.

Selon la figure 1, l'appareil comporte également un récepteur optique latéral monté à proximité de la cellule de mesure 4, à la partie amont de celle-ci.

Un hublot en verre 10" similaire aux hublots 10 et 10' permet à la lumière diffusée à la partie amont de la cellule de mesure 4 d'atteindre le récepteur latéral 13.

La lumière polarisée sortant du polariseur 9 et la lumière diffusée sortant par le hublot 10" sont respectivement concentrées sur des guides de lumière 15, 15' par des lentilles 14, 14' avant de parvenir aux récepteurs 7, 13.

L'unité de refroidissement, le capteur de température 2 ainsi que le récepteur longitudinal 7 et le récepteur latéral 13 sont reliés à des moyens de calcul et d'affichage programmables non représentés qui pilotent le test en conformité avec la norme à respecter.

A cet effet les moyens de calcul et d'affichage programmables commandent l'unité de refroidissement de la chambre cryostatée 1 en fonction des informations qui leur sont transmises par le capteur de température 2 et par les récepteurs 7, 13, et établissent parallèlement la courbe de détection représentant les variations de l'intensité lumineuse reçue par le récepteur longitudinal 7 et la courbe d'opacité représentant les variations de l'intensité lumineuse reçue par le récepteur latéral 13.

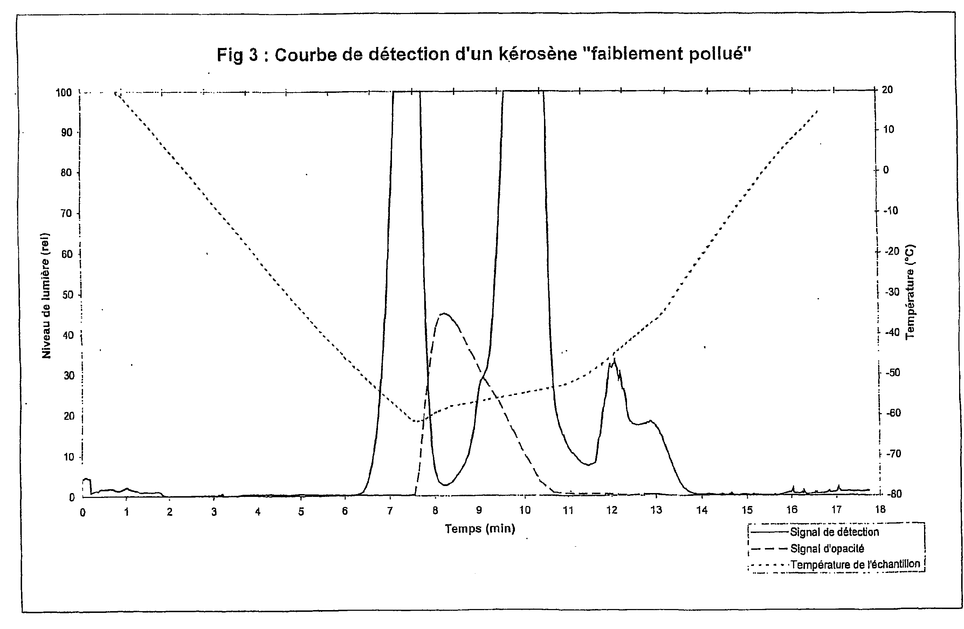

Les figures 3, 4 et 5 représentent trois exemples de telles courbes correspondant à trois échantillons différents de kérosène.

De manière plus précise, sur ces trois courbes, le temps exprimé en minutes est reporté en abscisse, alors que l'intensité lumineuse reçue par les récepteurs exprimée selon une graduation relative de 0 à 100 et la température de l'échantillon exprimée en degrés C sont reportées en ordonnée, respectivement sur l'échelle de gauche et sur l'échelle de droite.

Les courbes en pointillés représentent les variations de la température de l'échantillon en fonction du temps (échelle de droite).

Les courbes en tirets correspondent aux courbes d'opacité et représentent les variations en fonction du temps de l'intensité lumineuse reçue par le récepteur latéral (échelle de gauche).

Les courbes en traits pleins correspondent aux courbes de détection, et représentent les variations en fonction du temps de l'intensité lumineuse reçue par le récepteur longitudinal (échelle de gauche).

L'analyse de ces trois courbes permet de déterminer le point d'opacité, c'est-à-dire la température à partir de laquelle le sens de variation de la température dans la chambre cryostatée doit être inversé.

Les courbes en traits pleins permettent de déterminer le point de disparition des cristaux recherché.

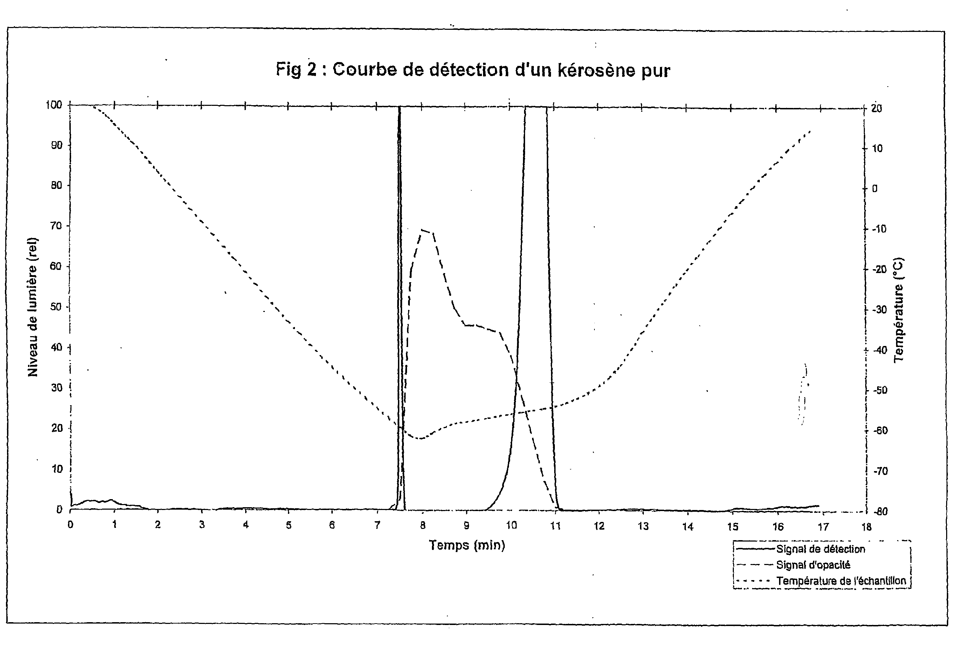

Selon la figure 2, dans le cas de kérosène pur non pollué, on a détecté l'apparition des premiers cristaux à 7 minutes 30 secondes, soit à une température de -59°C.

Le point d'opacité a été détecté à une température très proche de -60°C.

Le point de disparition des cristaux a été détecté à 11 minutes, soit à une température de -54°C.

Selon la figure 3, dans le cas d'un kérosène faiblement pollué, l'apparition des premiers cristaux a été détectée à 6 minutes 30 secondes, soit à une température de -45°C, et le point d'opacité à 7 minutes 45 secondes, soit à une température de -60°C.

La disparition du trouble dans l'échantillon a été détectée à 11 minutes 30 secondes, soit à une température de -55°C, et le point de disparition des cristaux à 13 minutes 30 secondes, soit à une température de -38,7°C.

Le « rebond » constaté sur la courbe de détection vers 12 minutes ne semble pas lié au matériel utilisé, mais plutôt à des phénomènes physiques au sein de l'échantillon.

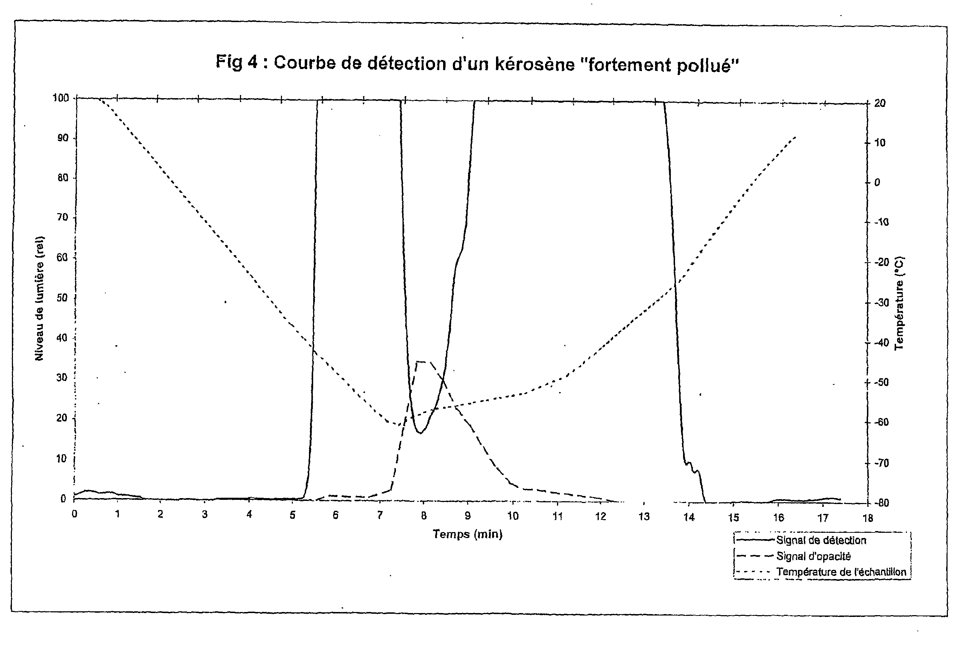

Selon la figure 4, dans le cas d'un kérosène fortement pollué, le point de disparition des cristaux a été détecté à 14 minutes 30 secondes, soit à une température de -27,5°C.

## Revendications

1. Procédé d'analyse d'un échantillon comportant les étapes suivantes :
- on monte un émetteur laser (6) ainsi qu'un récepteur optique longitudinal associé (7) de part et d'autre d'une cellule de mesure tubulaire (4) horizontale placée dans une chambre cryostatée (1) équipée d'un capteur de température (2) relié à des organes de réfrigération et de réglage de la température de sorte que le faisceau optique (8) émis par l'émetteur laser (6) soit aligné sur l'axe longitudinal de la cellule de mesure (4) et sur le récepteur optique longitudinal (7),
- on relie le capteur de température (2) les organes de réfrigération et de réglage de la température ainsi que le récepteur optique longitudinal (7) à des moyens de calcul et d'affichage programmables,
- on monte un diaphragme (12) directement en aval de l'émetteur laser (6) de sorte que le faisceau optique (8) émis par celui-ci soit suffisamment fin pour exclure toute réflexion sur les parois de la cellule de mesure (4),
- on monte, en amont du récepteur optique longitudinal (7), un polariseur (9) réglé de façon à ne pas pouvoir transmettre le faisceau optique directement émis par émetteur laser (6),
et **caractérisé en ce qu'**il comporte les étapes suivantes:
- on monte à proximité de la cellule de mesure (4), à la partie amont de celle-ci, un récepteur optique latéral (13) relié au faisceau optique (8) émis par l'émetteur laser (6) et aux moyens de calcul et d'affichage programmables,
- on recueille un échantillon d'un produit pétrolier tel qu'un kérosène, destiné à l'aviation,
- on introduit cet échantillon dans la cellule de mesure (4),
- on branche l'émetteur laser (6), le récepteur optique longitudinal (7) et le récepteur optique latéral (13) de façon à faire passer un faisceau optique au travers de l'échantillon,
- on abaisse graduellement la température de la chambre cryostatée (1) en enregistrant la courbe représentant les variations de l'intensité lumineuse reçue par le récepteur optique longitudinal (7) en fonction de la température ou courbe de détection, et la courbe représentant les variations de l'intensité lumineuse reçue par ce récepteur optique latéral (13) en fonction de la température ou courbe d'opacité et on détermine en utilisant cette dernière courbe la température de fin de cristallisation de l'échantillon ou point d'opacité à partir de laquelle on augmente à nouveau graduellement la température de cette chambre (1) et en poursuivant l'enregistrement de la courbe de détection et de la courbe d'opacité,
- on détermine à partir de la courbe de détection le point de disparition des cristaux de l'échantillon dans une gamme de température de - 5 à - 120°C, et
- on en déduit si le produit pétrolier est pur ou pollué, notamment par du gasoil

2. Procédé selon la revendication 1,
**caractérisé en ce qu'**
on utilise un dispositif comportant :
- une chambre cryostatée (1) équipée d'un capteur de température (2) relié à des organes de réfrigération et de réglage de la température,
- une tubulure de mesure (3) essentiellement en forme de U montée à la partie interne de la chambre cryostatée (1) et dont la branche centrale, essentiellement horizontale, constitue la cellule de mesure (4) tandis que les branches latérales (5, 5') permettent l'introduction de l'échantillon à analyser dans cette cellule ainsi que son extraction,
- un émetteur laser (6) et un récepteur optique longitudinal (7) associé, alignés de part et d'autre de la cellule de mesure (4), le long de l'axe longitudinal de celle-ci,
- un diaphragme (12) monté directement en aval de l'émetteur laser (6),
- un polariseur (9), monté en amont du récepteur optique longitudinal (7),
- des moyens de calcul et d'affichage programmables reliés au capteur de température (2), aux organes de réfrigération et de réglage de la température, ainsi qu'au récepteur optique longitudinal (7), et
- un récepteur optique latéral (13) monté à proximité de la cellule de mesure (4), à la partie amont de celle-ci, et relié aux moyens de calcul et d'affichage programmables.

3. **Procédé selon la revendication 2,**
**caractérisé en ce que**
l'intensité lumineuse est transmise au(x) récepteur(s) optique(s) (7, 13) par l'intermédiaire de guides de lumière (15, 15').

4. Procédé selon la revendication 3,
**caractérisé en ce que**
les guides de lumière (15, 15') coopèrent avec des lentilles (14, 14') susceptibles de concentrer le faisceau optique (8).

5. Procédé selon l'une quelconque des revendications 2 à 4,
**caractérisé en ce que**
la tubulure de mesure (3) est constituée par un élément métallique, notamment réalisé en aluminium équipé de hublots (10, 10', 10") permettant le passage du faisceau optique (8) à détecter.

6. Procédé selon l'une quelconque des revendications 2 à 5,
**caractérisé en ce que**
les organes de réfrigération et de réglage de la température sont constitués par une unité de refroidissement notamment à cycle de Stirling dont le doigt froid est équipé à son extrémité libre d'organes de transmission de la chaleur à contact sec coopérant avec la chambre cryostatée (1) de façon à permettre de la refroidir à la température souhaitée.

7. Procédé selon la revendication 6,
**caractérisé en ce qu'**
il est constitué par un appareil compact portatif.

## Claims

1. Method of analysis of a sample comprising the following stages:
- a laser emitter (6) and an associated longitudinal optical receiver (7) are mounted on either side of a horizontal tubular measurement cell (4) placed in a cryostatic chamber (1) equipped with a temperature sensor (2) connected to units for refrigeration and for temperature control in such a way that the light beam (8) emitted by the laser emitter (6) is aligned on the longitudinal axis of the measurement cell (4) and on the longitudinal optical receiver (7),
- the temperature sensor (2), the units for refrigeration and temperature control, and the longitudinal optical receiver (7) are connected to programmable means for calculation and display,
- a diaphragm (12) is mounted directly downstream of the laser emitter (6) in such a way that the light beam (8) emitted by the latter is sufficiently fine to exclude any reflection on the walls of the measurement cell (4),
- a polarizer (9), adjusted so as not to be able to transmit the light beam directly emitted by the laser emitter (6), is mounted upstream of the longitudinal optical receiver (7),
and **characterized in that** it comprises the following stages:
- a lateral optical receiver (13) connected to the light beam (8) emitted by the laser emitter (6) and to the programmable means for calculation and display, is mounted close to the measurement cell (4), in the part upstream of the latter,
- a sample of a petroleum product such as aviation-grade kerosene is collected,
- said sample is placed in the measurement cell (4),
- the laser emitter (6), the longitudinal optical receiver (7) and the lateral optical receiver (13) are switched on, so as to cause a light beam to pass through the sample,
- the temperature of the cryostatic chamber (1) is lowered gradually, recording the curve representing the variations in luminous intensity received by the longitudinal optical receiver (7) as a function of the temperature or detection curve, and the curve representing the variations in luminous intensity received by the lateral optical receiver (13) as a function of the temperature or opacity curve, and using this last-mentioned curve, the temperature of the end of crystallization of the sample, or opacity point, is determined, after which the temperature of said chamber (1) is gradually increased again, and recording of the detection curve and of the opacity curve is continued,
- the point of disappearance of the crystals from the sample is determined from the detection curve over a temperature range from -5 to -120°C, and
- from this it is deduced whether the petroleum product is pure or contaminated, notably with gas oil.

2. Method according to Claim 1, **characterized in that** a device is used comprising:
- a cryostatic chamber (1) equipped with a temperature sensor (2) connected to units for refrigeration and temperature control,
- a measurement nozzle (3), essentially U-shaped mounted in the interior of the cryostatic chamber (1) and whose central arm, essentially horizontal, constitutes the measurement cell (4) whereas the side arms (5, 5') permit introduction of the test sample in said cell as well as its removal,
- a laser emitter (6) and an associated longitudinal optical receiver (7), aligned on either side of the measurement cell (4), along the longitudinal axis of the latter,
- a diaphragm (12) mounted directly downstream of the laser emitter (6),
- a polarizer (9), mounted upstream of the longitudinal optical receiver (7),
- programmable means for calculation and display connected to the temperature sensor (2), to the units for refrigeration and temperature control, and to the longitudinal optical receiver (7), and
- a lateral optical receiver (13) mounted close to the measurement cell (4), in the part upstream of the latter, and connected to the programmable means for calculation and display.

3. Method according to Claim 2, **characterized in that** the luminous intensity is transmitted to the optical receiver(s) (7, 13) via light guides (15, 15').

4. Method according to Claim 3, **characterized in that** the light guides (15, 15') interact with lenses (14, 14') that are able to concentrate the light beam (8).

5. Method according to any one of Claims 2 to 4, **characterized in that** the measurement nozzle (3) comprises a metallic element, notably made of aluminium equipped with ports (10, 10', 10") permitting passage of the light beam (8) to be detected.

6. Method according to any one of Claims 2 to 5, **characterized in that** the units for refrigeration and temperature control comprise a refrigeration unit notably with a Stirling cycle in which the cold finger is equipped at its free end with dry-contact heat transmission devices interacting with the cryostatic chamber (1) enabling the latter to be cooled to the desired temperature.

7. Method according to Claim 6, **characterized in that** it comprises a compact, portable apparatus.

## Patentansprüche

1. Verfahren zur Analyse einer Probe mit den folgenden Schritten:
- Anordnen eines Laserlichtemitters (6) sowie eines dazugehörigen optischen Längsempfängers (7) beiderseits einer rohrförmigen Meßzelle (4), die horizontal in einer Kryostatkammer (1) angeordnet ist, die mit einem Temperaturaufnehmer (2) ausgestattet ist, der mit Kühl- und Temperaturregelungselementen verbunden ist, so daß der von dem Laserlichtemitter (6) ausgesandte Lichtstrahl (8) auf der Längsachse der Meßzelle (4) und auf den optischen Längsempfänger (7) ausgerichtet ist,
- Verbinden des Temperaturaufnehmers (2), der Kühl- und Temperaturregelungselemente sowie des optischen Längsempfängers (7) mit programmierbaren Rechner- und Anzeigeeinrichtungen,
- Anordnen einer Blende (12) direkt nach dem Laserlichtemitter (6), so daß der von diesem ausgesandte Lichtstrahl (8) ausreichend fein ist, um jede Reflexion an den Wänden der Meßzelle (4) auszuschließen,
- Anordnen eines Polarisators (9) vor dem optischen Längsempfänger (7), wobei der Polarisator so eingestellt ist, daß er den von dem Laserlichtemitter (6) direkt ausgesandten Lichtstrahl nicht hindurch lassen kann,
**dadurch gekennzeichnet, daß** es die folgenden Schritte aufweist:
- Anordnen eines optischen Seitenempfängers (13) in der Nähe der Meßzelle (4) an ihrem vorderen Teil, wobei der optische Seitenempfänger (13) mit dem von dem Laserlichtemitter (6) ausgesandten Lichtstrahl und mit programmierbaren Rechner- und Anzeigeeinrichtungen verbunden ist,
- Sammeln einer Probe eines für den Flugverkehr vorgesehenen Ölprodukts, wie beispielsweise Kerosin,
- Einführen dieser Probe in die Meßzelle (4),
- Einschalten des Laserlichtemitters (6), des optischen Längsempfängers (7) und des optischen Seitenempfängers (13), so daß ein Lichtstrahl durch die Probe hindurchgeht,
- allmähliches Absenken der Temperatur der Kryostatkammer (1) unter Aufzeichnen der Kurve, die die Veränderungen der von dem optischen Längsempfänger (7) empfangenen Lichtstärke in Abhängigkeit der Temperatur darstellt, oder der Detektionskurve, und der Kurve, die die Änderungen der von diesem optischen Seitenempfänger (13) empfangenen Lichtstärke in Abhängigkeit der Temperatur darstellt, oder der Opazitätskurve, und Bestimmen der Kristallisationsendtemperatur der Probe oder des Opazitätspunkts unter Verwendung dieser letzteren Kurve, von dieser Temperatur ab erneutes allmähliches Erhöhen der Temperatur dieser Kammer (1) unter Fortsetzung der Aufzeichnung der Detektionskurve und der Opazitätskurve,
- Bestimmen des Verflüchtigungspunkts der Kristalle der Probe unter Verwendung der Detektionskurve in einem Temperaturbereich von -5 bis - 120°C, und
- Herleiten, ob das Erdölprodukt rein oder, insbesondere durch Diesel, verunreinigt ist.

2. Verfahren nach Anspruch 1, **dadurch gekennzeichnet, daß** eine Vorrichtung verwendet wird, die aufweist:
- eine Kryostatkammer (1), die mit einem Temperaturaufnehmer (2) ausgestattet ist, der mit Kühl- und Temperaturregelungselementen verbunden ist,
- eine im Innern der Kryostatkammer (1) angeordnete, im wesentlichen U-förmige Meßzelle (3), deren zentraler im wesentlichen horizontaler Zweig die Meßzelle (4) bildet, während die seitlichen Zweige (5,5') die Einführung der zu analysierenden Probe in diese Zelle sowie deren Entnahme erlauben,
- einen Laserlichtemitter (6) und einen dazugehörigen optischen Längsempfänger (7), die beiderseits der Meßzelle (4) entlang deren Längsachse angeordnet sind,
- eine direkt nach dem Laserlichtemitter (6) angeordnete Blende (12),
- einen vor dem optischen Längsempfänger (7) angeordneten Polarisator (9),
- programmierbare Rechner- und Anzeigeeinrichtungen, die mit dem Temperaturaufnehmer (2), den Kühl- und Temperaturregelungselementen sowie mit dem optischen Längsempfänger (7) verbunden sind, und
- einen optischen Seitenempfänger (13), der in der Nähe der Meßzelle (4) an deren vorderen Teil angeordnet ist und mit den programmierbaren Rechner- und Anzeigeeinrichtungen verbunden ist.

3. Verfahren nach Anspruch 2, **dadurch gekennzeichnet, daß** die Lichtstärke über Lichtleiter (15,15') zu dem oder den optischen Empfänger(n) (7,13) übertragen wird.

4. Verfahren nach Anspruch 3, **dadurch gekennzeichnet, daß** die Lichtleiter (15,15') mit Linsen (14,14') zusammenwirken, die den optischen Strahl (8) fokussieren können.

5. Verfahren nach einem der Ansprüche 2 bis 4, **dadurch gekennzeichnet, daß** das Meßrohr (3) aus einem aus Metall, insbesondere Aluminium, hergestellten Element besteht, das mit Fenstern (10,10',10") versehen ist, die den Durchgang des zu detektierenden optischen Strahls (8) erlauben.

6. Verfahren nach einem der Ansprüche 2 bis 5, **dadurch gekennzeichnet, daß** die Kühl- und Temperaturregelungseinrichtungen aus einer Kühleinheit, insbesondere mit Stirling-Zyklus, bestehen, deren Kühlfinger an seinem freien Ende mit Trockenkontakt-Wärmedurchlaßelementen ausgestattet ist, die mit der Kryostatkammer (1) zusammenwirken, um zu erlauben, sie auf die gewünschte Temperatur zu kühlen.

7. Verfahren nach Anspruch 6, **dadurch gekennzeichnet, daß** die Vorrichtung durch ein kompaktes tragbares Gerät gebildet ist.
